# FASCICULE DE BREVET EUROPEEN

(11) **EP 1 059 088 B1**
(45) Date de publication et mention de la délivrance du brevet: **29.09.2004**
(21) Numéro de dépôt: 00401601.0
(22) Date de dépôt: 07.06.2000
(51) Int. Cl.: A61K 39/395, C07K 16/42, C07K 16/06, C07K 16/18, C07K 1/22, A61P 37/06

(54) **Procédé de purification des fractions d'Ig connectées possédant une activité immunomodulatrice**
Reinigungsverfahren von verbindeten Ig Fraktionen mit immunomodulierender Aktivität
Ig connected fraction purification process, the Ig modulating the immune response

(30) Priorité: 07.06.1999 FR 9907153; 29.12.1999 FR 9916632
(43) Date de publication de la demande: 13.12.2000
(73) Titulaire: Laboratoire Français du Fractionnement et des Biotechnologies, 91940 Les Ulis (FR)
(72) Inventeur: Bourel, Dominique, 59110 La Madeleine (FR); Bruley-Rosset, Martine, 94210 La Varenne (FR); Dhainaut, Frédéric, 91870 Boissy le Sec (FR); Lirochon, Jacky, 91650 Breuillet (FR)
(74) Mandataire: Warcoin, Jacques

(56) Documents cités:
- DIETRICH G ET AL: "A V region-connected autoreactive subfraction of normal human serum immunoglobulin G." EUROPEAN JOURNAL OF IMMUNOLOGY, (1992 JUL) 22 (7) 1701-6., XP000877158
- JORDAN S C ET AL: "Posttransplant therapy using high-dose human immunoglobulin ( intravenous gammaglobulin) to control acute humoral rejection in renal and cardiac allograft recipients and potential mechanism of action." TRANSPLANTATION, (1998 SEP 27) 66 (6) 800-5., XP000877173
- PACHECO-GARCIA U ET AL: "Altered pattern of connectivity in serum immunoglobulins from pemphigus vulgaris patients." SCANDINAVIAN JOURNAL OF IMMUNOLOGY, (1999 APR) 49 (4) 424-30., XP000877174
- FLAN B.: "[Fractionation technique and biochemical properties of IV Ig]. TECHNIQUE DE FRACTIONNEMENT ET PROPRIETES BIOCHIMIQUES DES IMMUNOGLOBULINES INTRAVEINEUSES (IGIV)." SANG THROMBOSE VAISSEAUX, (OCT. 1999) 11/SPEC. ISS. (45-51). , XP000939133

## Description

La présente invention concerne un procédé de préparation de fractions d'lg à partir d'Immunoglobulines intraveineuses polyvalentes humaines (IgIV) qui seraient plus particulièrement responsables de l'effet immunomodulateur observé au cours des traitements de certaines maladies autoimmunes. L'invention porte sur des fractions d'lg qui possèdent une réactivité vis-à-vis des IgM, des F(ab')2 d'IgG ou de l'haptène DNP et pas ou peu de réactivité vis-à-vis des antigènes du non-soi, c'est à dire des fractions d'lg qui présentent des interactions de type idiotypique entre elles (fraction connectée) ou qui comprennent des anticorps naturels réagissant avec l'haptène DNP. Ces fractions montrent une polyréactivité vis-à-vis d'autoantigènes donnés.

Les préparations d'IgIV sont utilisées depuis de nombreuses années pour le traitement de multiples états pathologiques. Les indications majeures peuvent être regroupées en trois cibles thérapeutiques :
- Les déficits immunitaires primitifs ou secondaires,
- Le traitement de certaines maladies autoimmunes,
- Les complications infectieuses et la maladie du greffon contre l'hôte après greffes de cellules hématopoïétiques allogéniques.

Dans le cas des déficits immunitaires, les IglV constituent un traitement substitutif qui permet d'apporter des IgG dont la concentration plasmatique chez les malades n'est pas suffisante pour neutraliser le développement d'infections virales ou bactériennes.

Pour les maladies autoimmunes, l'efficacité des IgIV est liée à des effets immunomodulateurs complexes. Prescrites dans le cadre des greffes de moelle osseuse, les IglV correspondent à un traitement substitutif en attendant la reconstitution immunologique des sujets greffés et exercent un effet immunomodulateur pour la maladie du greffon contre l'hôte.

Les IglV sont préparées à partir d'un pool de plasmas provenant de plusieurs milliers de donneurs, elles présentent une répartition en sous-classes et des spécificités anticorps reflétant celle de la population générale. Ainsi, les IglV peuvent être considérées comme un produit contenant la totalité du répertoire des anticorps naturels et des anticorps dirigés contre les antigènes extérieurs et les autoantigènes.

Le concept d'immunorégulation par les IglV s'est largement développé depuis la démonstration de leur efficacité dans le purpura thrombopénique auto-immun (PTAI) en 1981 (1). Les IglV ont été par la suite utilisées dans de nombreuses pathologies autoimmunes ou inflammatoires. Certaines indications, pour lesquelles l'efficacité des IglV a été clairement établie, sont officiellement reconnues par les instances réglementaires. Il s'agit du PTAI, de la maladie de Kawasaki où elles préviennent très efficacement les complications anévrismales (2,3), de la greffe de cellules hématopoïétiques allogéniques où elles modulent la réaction du greffon contre l'hôte (4) et plus récemment, de la rétinochoroïdite de Birdshot, où elles améliorent l'acuité visuelle et où elles permettent parfois de réduire la corticothérapie (5).
D'autres indications sont considérées par les experts comme étant justifiées. Certaines cytopénies par exemple où les IglV entraînent une amélioration rapide, mais souvent transitoire (6) et les hémophilies avec inhibiteurs (autoanticorps anti-facteur VIII) où en revanche l'amélioration peut être durable (7,8). Des résultats contradictoires ont été obtenus dans les avortements à répétition avec des taux de succès encourageants dans certaines séries (9,10).

Depuis une dizaine d'années, les IgIV ont connu un essor très important en neurologie grâce à des études multicentriques contrôlées avec des critères d'efficacité à la fois quantitatifs (scores neurologiques) et qualitatifs (nombre de patients améliorés). Ainsi, dans la maladie de Guillain-Barré de l'adulte, les IglV sont aussi efficaces que les échanges plasmatiques et sont mieux tolérées (11,12). Elles sont conseillées en première intention dans les formes de l'enfant (13). Elles sont plus efficaces versus placebo dans les polyneuropathies inflammatoires chroniques démyelinisantes (14) et dans les dermatomyosites (15). Elles sont aussi efficaces et mieux tolérées que les échanges plasmatiques au cours des crises aiguës de myasthénie (16). Enfin, une étude versus placebo a démontré l'efficacité des IglV dans les formes alternant rémissions et poussées de la sclérose en plaques (17).

Plusieurs mécanismes ont été proposés pour expliquer la diversité d'action des IgIV (18):
- Le blocage des récepteurs Fc à la surface des macrophages, monocytes, neutrophiles et eosinophiles.
- La neutralisation des autoanticorps circulants par des anticorps anti-idiotype,
- L'inhibition des effets néfastes dus à l'activation du complément,
- La modulation du réseau des cytokines,
- et/ou la sélection des répertoires immunitaires par interaction avec les lymphocytes T et B.

Ces mécanismes pourraient rendre compte des effets à la fois précoces et prolongés des IgIV.

Une fraction (dite connectée) d'lg peut être purifiée sur une colonne d'affinité dont les F(ab')2 d'IgIV ont été couplés à des billes de Sepharose (19, 20 et 25). Les IgM contenus dans le sérum d'individus normaux se lient aux fragments F(ab')2 des IgG autologues et inhibent l'association de ces IgG aux autoantigènes (21). Les IgM contribuent à réguler l'autoréactivité naturelle des IgG à travers des interactions de type idiotypique (21). Ces Ig ou leur F(ab')2 peuvent inhiber la liaison de certains autoanticorps à leurs antigènes comme cela a été démontré par des tests réalisés in vitro (22). La fraction connectée d'lg obtenue à partir d'IgIV renfermerait en particulier des anticorps reconnaissant des déterminants anti-idiotypiques présents sur les autoanticorps IgG ou IgM capables de se neutraliser entre eux et d'altérer la fonction et la dynamique du réseau idiotypique (23). Par ailleurs, une fraction d'Ig caractérisée en ce qu'elle réagit avec l'haptène DNP est décrite comme contenant des anticorps naturels polyréactifs et autoréactifs (24).

D'autres documents décrivent le principe général pour l'obtention de fractions connectées. Parmi ces documents, on peut citer la demande de brevet WO 98 /26086 qui porte sur un procédé pour préparer une composition purifiée d'anticorps comprenant des anticorps anti-idiotypes, ledit procédé consistant en l'adsorption d'un pool d'IgG sur un substrat solide contenant un déterminant idiotypique d'un autoanticorps, et en une élution.
EP 293 606 décrit une méthode générale pour la purification d'un anticorps X par interaction idiotypique / anti-idiotypique comprenant les étapes suivantes :
a) attachement d'un anticorps Y à un support solide, ledit anticorps reconnaissant l'idiotype de X,
b) mise en contact d'un échantillon contenant un anticorps X avec le support solide dans un tampon approprié,
c) élution et d) récupération de l'anticorps X purifié.

WO 97/19113 a trait à l'utilisation d'anticorps monoclonaux anti-idiotypes de type IgG comme immunorégulateur de la réponse immunitaire, en particulier pour le traitement des maladies autoimmunes.

Actuellement, la tolérance et l'efficacité des IgG polyvalentes mises dans le commerce, notamment TEGELINE® (LFB, France) sont reconnues en particulier dans le traitement du PTI, de la maladie de Kawasaki et de la rétinochoroïdite de type "Birdshot", pathologies pour lesquelles des AMM ont été obtenues. Cependant, les posologies actuelles dans ces indications sont importantes, et le mode d'administration reste lourd et complexe (perfusions de plusieurs heures en milieu hospitalier). Le problème consiste donc à préparer une fraction active dans les pathologies autoimmunes, de façon à rendre la préparation plus efficace et d'une utilisation plus commode.

L'objectif à la base de la présente invention est donc d'obtenir des Ig spécifiques permettant une diminution des posologies, ayant une efficacité maintenue voire accrue, une meilleure tolérance, et dont le mode d'administration est plus simple. On a montré qu'il est possible de préparer des fractions répondant aux problèmes mentionnés précédemment en les préparant à partir de pools d'Ig de manière à ce qu'elles aient une réactivité anti IgM, anti F(ab')2 d'IgG ou anti DNP, peu ou pas de réactivité vis-à-vis des antigènes du non-soi, et/ou qui montrent une polyréactivité vis-à-vis de certains autoantigènes.

### Description

Ainsi, la présente invention concerne la purification des Ig contenues dans les IgIV polyvalentes qui seraient plus particulièrement responsables de l'effet immunomodulateur observé au cours du traitement de certaines maladies autoimmunes. L'invention repose sur les caractéristiques de ces fractions d'IgG qui possèdent une réactivité vis-à-vis des IgM, des F(ab')2 d'IgG ou de l'haptène DNP et pas ou peu vis-à-vis de l'anatoxine tétanique et de l'antigène HBs (antigènes du non-soi), c'est à dire des fractions comportant des Ig présentant des interactions de type idiotypique entre elles (fraction connectée) ou comprenant des anticorps naturels. Ces fractions montrent une polyréactivité vis-à-vis de certains autoantigènes.

La préparation des fractions d'lg est faite par chromatographie d'affinité en utilisant la propriété de ces Ig de se reconnaître entre elles, de reconnaître les IgM ou de se lier à l'haptène DNP. La matière première utilisée pour obtenir ces fractions provient d'Ig polyvalentes, notamment celles qui sont préparées et commercialisées par le LFB (France), ou de toute autre fraction intermédiaire obtenue au cours du procédé de fabrication des IgIV polyvalentes à usage thérapeutique. Le procédé général de préparation d'IgIV polyvalentes comporte essentiellement les étapes suivantes :
- Fractionnement du plasma provenant d'un pool de donneurs par précipitation, adsorption et/ou filtration puis ultrafiltration (obtention d'une première fraction " PSO 1"),
- Traitement par la pepsine à pH acide, formulation, répartition, et lyophilisation (obtention du produit TEGELINE® ),
- Un autre traitement pourrait utiliser la chromatographie sur colonne échangeuse d'anions, ultrafiltration, obtention d'une fraction intermédiaire (appelée " PSO 2") et chauffage, ultrafiltration, formulation et répartition (obtention d'une fraction IgIV liquide).
On entend par " Ig polyvalentes " dans le cadre de l'invention, des IgG ou des IgM polyvalentes entières, des fragments d'IgG polyvalentes tels que F(ab')2 ou F(ab) et toute fraction intermédiaire obtenue au cours du procédé de fabrication des IgIV polyvalentes.

Un premier aspect de l'invention concerne une fraction d'lg réagissant avec au moins un composant sélectionné parmi les IgM, les F(ab')2 d'IgG et l'haptène DNP avec un taux d'enrichissement supérieur à 20 par rapport à l'activité des Ig polyvalentes initiales, et en ce qu'elle ne réagit pas avec l'anatoxine tétanique et l'antigène HBs avec un taux d'enrichissement inférieur à 5 par rapport à l'activité des Ig polyvalentes initiales.

Cette fraction d'lg peut consister en une fraction d'IgG ou une fraction d'IgM.
De préférence, elle réagit avec un composant sélectionné parmi les IgM, les F(ab')2 d'IgG et l'haptène DNP avec un taux d'enrichissement supérieur à 40 par rapport à l'activité des Ig polyvalentes initiales.

La fraction selon l'invention peut également réagir avec au moins l'un des autoantigènes sélectionnés parmi la myosine, l'actine, la tubuline, et la protéine basique de la myéline (MBP) avec un taux d'enrichissement supérieur à 10, de préférence 20 par rapport à l'activité des Ig polyvalentes initiales.

Avantageusement, la fraction réagit avec l'ensemble des autoantigènes évoqués précédemment.

Une fraction préférée selon l'invention peut se définir en ce qu'elle réagit avec un composant sélectionné parmi les IgM, les F(ab')2 d'IgG et l'haptène DNP avec un taux d'enrichissement supérieur à 40 par rapport à l'activité des Ig polyvalentes initiales, et avec la myosine, l'actine, la tubuline, et la MBP avec un taux d'enrichissement moyen supérieur à 20 par rapport à l'activité des lg polyvalentes initiales.

Les fractions évoquées peuvent réagir avec les IgM ou les F(ab')2 d'IgG. Elles peuvent aussi réagir avec l'haptène DNP et dans ce cas, elle ne réagissent pas avec les IgM et les F(ab')2 d'IgG.

Un deuxième aspect de l'invention porte sur un procédé de préparation de fractions d'Ig caractérisé en ce qu'il comprend les étapes suivantes :
a) Préparation d'un support insoluble sur lequel sont greffées un composant sélectionné parmi les IgG,les IgM polyvalentes et du DNP-Lysine,
b) adsorption d'lg polyvalentes sur le support obtenu à l'étape a),
c) élution des Ig retenues sur la partie des immunoglobulines liées au support de façon à recueillir la fraction connectée par interactions idiotypiques IgG-IgG ou IgM-IgG, ou élution de la fraction interagissant avec le DNP,
d) sélection des fractions présentant une réactivité vis-à-vis des IgM, des F(ab')2 d'IgG ou de l'haptène DNP, pas ou peu de réactivité vis-à-vis d'antigènes du non-soi et/ou une polyréactivité vis-à-vis d'autoantigènes donnés,
e) sélection des fractions présentant une activité inhibitrice de la prolifération des lymphocytes en culture mixte, de préférence avec une efficacité 10 à 50 fois supérieure à TEGELINE®.

Dans ce procédé, les Ig absorbées peuvent être des IgG ou des IgM.

Les fractions d'lg obtenues sont préparées à partir d'Ig polyvalentes ou tout autre fraction intermédiaire obtenue au cours du procédé de fabrication des IglV à usage thérapeutique. Ces Ig polyvalentes peuvent être des IgG ou des IgM.
Dans les Ig polyvalentes, il existe des anticorps naturels qui interagissent avec l'haptène DNP et des anticorps qui interagissent avec les idiotypes exprimés par des autoanticorps de type IgG ou IgM (fraction connectée) et qui possèdent une certaine autoréactivité. On entend par " fraction connectée " dans le cadre de l'invention une fraction qui présente un pourcentage élevé d'Ig interagissant entre elles ou avec des IgG ou des IgM par liaison idiotype - anti-idiotype.

La stratégie utilisée pour déterminer, parmi les différentes fractions, la ou les fraction(s) possédant les propriétés recherchées, c'est à dire, les fractions contenant le plus fort titre en autoréactivité et réagissant avec le plus grand nombre d'autoantigènes, consiste à les soumettre à un criblage pouvant comporter plusieurs étapes successives.

Les différents tests in vitro et/ou in vivo utilisés permettent de sélectionner à chaque étape les fractions les plus actives sur des critères de plus en plus spécifiques.

Le procédé selon l'invention peut donc comporter des étapes permettant la sélection de fractions d'Ig présentant des caractéristiques données.

En ce sens, l'étape d) peut comprendre une mesure du taux d'enrichissement en anticorps réactifs contre les IgM, les F(ab')2 des IgG ou l'haptène DNP utilisées pour la purification.
L'étape d) peut également comporter une mesure de la réactivité pour l'anatoxine tétanique et l'antigène HBs en prenant le taux d'enrichissement comme valeur contrôle.
De préférence, l'étape d) comprend un test ELISA effectué sur un panel d'autoantigènes sélectionnés notamment parmi l'actine, la myosine, la MBP, et la tubuline.

L'étape d) du procédé selon l'invention peut comprendre en outre un test de compétition pour contrôler l'activité neutralisante des fractions vis-à-vis d'autoanticorps provenant de sérum de patients atteints de maladies autoimmunes, et/ou un test d'inhibition de la réaction lymphocytaire mixte avec des cellules humaines pour mesurer la capacité inhibitrice.
Ce test de réaction lymphocytaire mixte peut comprendre les étapes suivants :
- obtention de prélèvements sanguins d'un donneur A et d'un donneur B incompatible au niveau des antigènes du complexe majeur d'histocompatibilité (CMH),
- purification des cellules mononuclées sur ficoll,
- mise ne culture de 2.10⁵ cellules du donneur B en présence de 210⁵ cellules du donneur A,
- mesure de la prolifération des cellules au jour 4 par mesure de l'incorporation de thymidine tritiée.

L'étape a) consiste en un greffage d'IgG ou d'IgM polyvalentes ou de DNP-Lysine sur un support insoluble, notamment sur un gel de Sepharose®, de Trisacryl®, d'Affiprep®, d'Affigel® , gels activés avec les groupements CNBr, NHS ou C₅H₈O₂ (glutaraldéhyde). Les lg déposées sur le support solide obtenu à l'étape a) sont adsorbées soit sous forme d'Ig polyvalentes lyophilisées et remises en solution ou sous forme liquide, soit sous forme de fractions intermédiaires obtenues au cours d'un procédé de fabrication des Ig polyvalentes. Les lg déposées comprennent des IgG ou des IgM.
Avantageusement, l'absorption est effectuée dans des conditions de température allant de 4° à 40°C et en tampon phosphate 20 mM ou équivalent comprenant du NaCI dont la concentration peut varier de 0 M à 3 M.
L'élution des Ig retenues à l'étape b) sont de préférence éluées à l'étape c) avec un tampon d'ions dissociant la liaison Ag-Ac ou DNP-Ac, sélectionné notamment parmi les chaotropes tels que la glycine-HCl ou l'iodure de sodium (Nal) dans des conditions faisant varier le pH, de préférence entre 2,8 à 4,0.

Dans un mode particulier de réalisation, ce procédé comprend les étapes suivantes :
a) Greffage d'IgG ou d'IgM polyvalentes ou de DNP-Lysine sur un support solide ou support d'affinité (immunoadsorbant) habituellement utilisé en chromatographie d'affinité. De tels supports sont bien connus de l'homme du métier. On peut citer par exemple un gel de Sepharose®, de Trisacryl®, d'Affiprep®, d'Affigel®, gels activés avec les groupements CNBr, NHS ou C₅H₈O₂ (glutaraldéhyde).
b) Adsorption d'lg en tampon phosphate 20 mM ou équivalent comprenant du NaCI dont la concentration peut varier de 0 M à 3 M sur le support solide obtenu à l'étape a), déposées soit sous forme d'lg polyvalentes lyophilisées et remises en solution ou sous forme liquide, soit sous forme de fractions intermédiaires obtenues au cours du procédé de fabrication des Ig polyvalentes. Les lg adsorbées comprennent des IgG ou des IgM.
c) Elution des Ig retenues à l'étape b) avec un tampon d'ions dissociant la liaison Ag-Ac sélectionné notamment parmi les chaotropes tels que la glycine-HCl ou l'iodure de sodium (Nal) dans des conditions faisant varier le pH, de préférence entre 2.8 à 4.0, et/ou la force ionique et/ou par toute autre méthode équivalente permettant la rupture des liaisons IgG-IgG, IgG-IgM ou Ig-DNP-Lysine de façon à obtenir des fractions d'lgayant un profil de réactivité différent de celui des lg polyvalentes de départ.
d) Mesure en ELISA du taux d'enrichissement en anticorps réactifs contre des IgM, des F(ab')2 d'IgG ou l'haptène DNP ou TNP utilisés pour la purification, mesure de la réactivité pour l'anatoxine tétanique et l'antigène HBs en prenant le taux d'enrichissement comme valeur contrôle, et mesure du taux d'enrichissement en réactivité vis-à-vis d'un panel d'autoantigènes sélectionnés notamment parmi l'actine, la myosine, la MBP, et la tubuline.
Comme évoqué précédemment, on peut également inclure dans ce procédé une étape supplémentaire comprenant un test de réaction lymphocytaire.

Dans chaque cas, la fraction non retenue sur les différentes colonnes peut également servir de contrôle en plus de la préparation initiale d'Ig.
Bien entendu, certains paramètres du procédé peuvent être modifiés à la convenance de l'homme du métier par de simples expériences de routine. L'invention porte donc également sur un procédé évoqué ci-dessus dans lequel les paramètres sont déterminés en fonction des fractions que l'on a sélectionnée au préalable à l'étape d). Il s'agit de définir les paramètres optimums pour obtenir une fraction ayant les propriétés particulières que l'on souhaite et d'appliquer ensuite ces paramètres à l'échelle d'un procédé industriel selon l'invention. De tels paramètres peuvent être les paramètres qui caractérisent les fractions décrites ci-dessus. Ainsi, le procédé peut être adapté à l'obtention des fractions décrites ci-dessus. De même, l'invention vise un procédé de fabrication industrielle de fractions présentant une réactivité vis-à-vis d'un composant sélectionné parmi les IgM, les F(ab')2 d'IgG et l'haptène DNP, pas ou peu de réactivité vis-à-vis d'antigènes du non-soi et une polyréactivité vis-à-vis d'autoantigènes donnés caractérisé en ce qu'on met en oeuvre les étapes a) b) et c) décrite ci-dessus en respectant ou en adaptant les paramètres utilisés lors de la préparation des fractions d'intérêt préalablement sélectionnées.
L'invention a également pour objet les fractions susceptibles d'être obtenues à partir du procédé évoqué précédemment.

Les propriétés immunomodulatrices des quelques fractions sélectionnées par les tests in vitro peuvent être également déterminées in vivo dans plusieurs modèles animaux de maladies autoimmunes et de la maladie du greffon contre l'hôte (GVH) après allogreffes.

Plusieurs types de modèles ont été choisis en fonction du mécanisme d'action mis en jeu :
- Modèles dans lequel la fonction effectrice est assurée par l'intermédiaire de cellules T ou par des anticorps,
- Modèles dans lequel les mécanismes dépendent de l'interaction avec les F(ab)'2 ou les Fc.

Deux maladies autoimmunes expérimentales chez le rat, où la fonction effectrice est assurée par l'intermédiaire de cellules T, sont plus particulièrement choisies car elles ont été décrites comme étant sensibles à l'administration d'IgIV et présentent l'avantage de pouvoir apporter une réponse rapide quant à l'efficacité des fractions (les effets protecteurs sont évaluables en 4 semaines environ). Il s'agit des modèles suivants :
1) L'uvéite autoimmune expérimentale ou UAE induite par l'injection de l'antigène rétinal de boeuf ou de son peptide immunodominant à des rats Lewis.
2) La polyarthrite rhumatoïde (PR) induite chez des rats Dark Agouti par injection de collagène bovin de type II.

Dans chaque cas, la sévérité de la maladie est évaluée de manière clinique et/ou histopathologique et plusieurs paramètres biologiques tels que la perte de poids, la production d'anticorps contre l'autoantigène injecté sont mesurés au cours du temps.

Un modèle de GVH aiguë chez le rat a été ajouté car cette maladie a été décrite comme sensible à l'administration d'IgIV. La GVH est induite chez le rat hybride (Lewis× Brown-Norway) par injection de cellules lymphoïdes provenant de rats Lewis. La maladie est évaluée par la perte de poids, la présence d'erythème et le taux de mortalité.

Le modèle animal d'anémie hémolytique autoimmune (AHA) qui met principalement en jeu l'action des anticorps, est proche des pathologies hémolytiques observées chez l'homme. Elle est induite par l'injection de globules rouges (GR) de rat à des souris C3H préalablement splénectomisées. Ce test est utile en raison de l'efficacité des IgIV observée dans l'anémie hémolytique chez l'homme. Le développement de l'anémie est suivi par la diminution du nombre de GR et l'apparition dans le sérum des animaux d'autoanticorps dirigés contre leurs propres GR.

L'effet protecteur du produit TEGELINE®, IgG ou IgM polyvalentes ou tout autre produit intermédiaire obtenus au cours du procédé de fabrication des Ig polyvalentes, est préalablement testé dans ces modèles et les conditions optimales d'administration (dose, nombre, délais et voie d'injection) sont déterminées. Les fractions sélectionnées sont injectées à des doses de cinq à vingt fois inférieures à celles de TEGELINE® et l'efficacité de ces traitements est mesurée dans les différents modèles de maladies autoimmunes.

De surcroît, on peut mettre en oeuvre des modèles expérimentaux utilisant des cellules humaines.
- La souris SCID/NOD humanisée apparaît comme le meilleur modèle pour évaluer l'efficacité in vivo sur des cellules humaines pathologiques des fractions présélectionnées par les tests sur les modèles animaux.

Les modèles de la cirrhose biliaire primitive, de la myasténie et de la thyroïdite d'Hashimoto ont été sélectionnés car les cellules provenant de ces pathologies ont déjà été greffées avec succès à des souris SCID. D'autres pathologies pourront être choisies par la suite.

Dans une phase ultérieure et dans le but d'augmenter les connaissances sur le mécanisme d'action d'une fraction donnée, démontrée comme efficace, on peut mettre en oeuvre d'autres modèles complémentaires afin d'étendre les indications de l'utilisation des fractions dérivées des IgIV, telles que TEGELINE® ou autres.

L'étape d) peut donc comprendre en outre un ou plusieurs test(s) in vitro, notamment les tests décrits ci-dessus.

Ainsi, le procédé selon l'invention permet notamment la préparation et la sélection des fractions dont les caractéristiques sont définies ci-dessus.

Une fois que les fractions d'intérêt ont été identifiées, les paramètres des étapes a), b) et c) peuvent être employés dans le cadre d'un procédé industriel de fabrication desdites fractions. Un tel procédé avec les paramètres adéquats selon les fractions d'intérêt préalablement sélectionnées est un objet supplémentaire de l'invention.

Un aspect complémentaire de l'invention porte sur les fractions susceptibles d'être obtenues à partir du procédé défini ci-dessus.
Il est nécessaire de noter que la description de la présente invention n'est pas limitative, que des procédés équivalents et des fractions équivalentes forment également l'invention.

Les fractions selon l'invention présentent plusieurs avantages dont les principaux sont les suivants :
- Une diminution des posologies. Etant donné que le nouveau produit proposé correspond à une fraction contenue dans les Ig polyvalentes la quantité injectée d'lg présentant des propriétés immunomodulatrices est inférieure à celle des IgIV habituellement prescrites. Les doses efficaces peuvent être réduites d'un facteur de 5 à 20 voire plus. Cet avantage est considérable car les posologies actuellement utilisées pour les Ig polyvalentes disponibles sont très élevées : de l'ordre de 1 à 2 g/kg.
- Une efficacité maintenue voire accrue car le produit est enrichi en lg immunomodulatrices,
- Une meilleure tolérance. Avec des concentrations plus faibles, la tolérance du nouveau produit est améliorée. En effet, actuellement il est nécessaire de prendre certaines précautions au cours de l'administration des IgIV avec, en particulier, une perfusion lente du produit pendant plusieurs heures pour éviter certains effets secondaires, comme par exemple des réactions allergiques.
- Une prescription simplifiée. L'administration de faibles doses permet d'instaurer des traitements ambulatoires venant se substituer aux perfusions actuelles réalisées en milieu hospitalier.

Un aspect supplémentaire porte sur l'utilisation des fractions d'lg selon l'invention pour la préparation d'un médicament. Ce médicament est plus particulièrement adapté au traitement des maladies autoimmunes, de la GVH, et/ou du rejet de greffe après transplantation.

Les fractions selon l'invention sont utiles pour la préparation d'un médicament destiné au traitement de la maladie de Kawasaki, de la rétinochoroïdite de Birdshot, éventuellement en association avec une corticothérapie, pour le traitement de certaines cytopénies, des hémophilies avec inhibiteurs (autoanticorps anti-facteur VIII), et/ou pour prévenir ou empêcher le rejet immunitaire de greffes de cellules et/ou d'organes et le développement de la GVH après greffe de cellules allogéniques.

Les fractions selon l'invention sont également utiles pour la préparation d'un médicament destiné au traitement de maladies neurologiques, notamment la maladie de Guillain-Barré de l'adulte, les polyneuropathies inflammatoires chroniques démyelinisantes, les dermatomyosites, la myasthénie, et/ou la sclérose en plaques.

On se référera aux légendes des figures présentées ci-après pour la suite de la description.

### Légendes

**Figure 1A-1D:** Evaluation des propriétés d'une fraction obtenue à partir de TEGELINE® (support solide à base d'Affigel greffé avec TEGELINE®)
Les paramètres du procédé de préparation de cette fraction sont explicités plus en détail à l'exemple 1 ci-après.
FNA signifie fraction non absorbée.
Les figures 1A et 1C illustrent la réactivité spécifique vis-à-vis des F(ab')2 d'IgG et les figures 1B et 1D représentent la réactivité vis-à-vis des autoantigènes.

**Figure 2A-2D :** Evaluation des propriétés d'une fraction obtenue à partir de TEGELTNE® (support solide à base de NHS-Sepharose).
Les paramètres du procédé de préparation de cette fraction sont explicités plus en détail à l'exemple 2 ci-après.

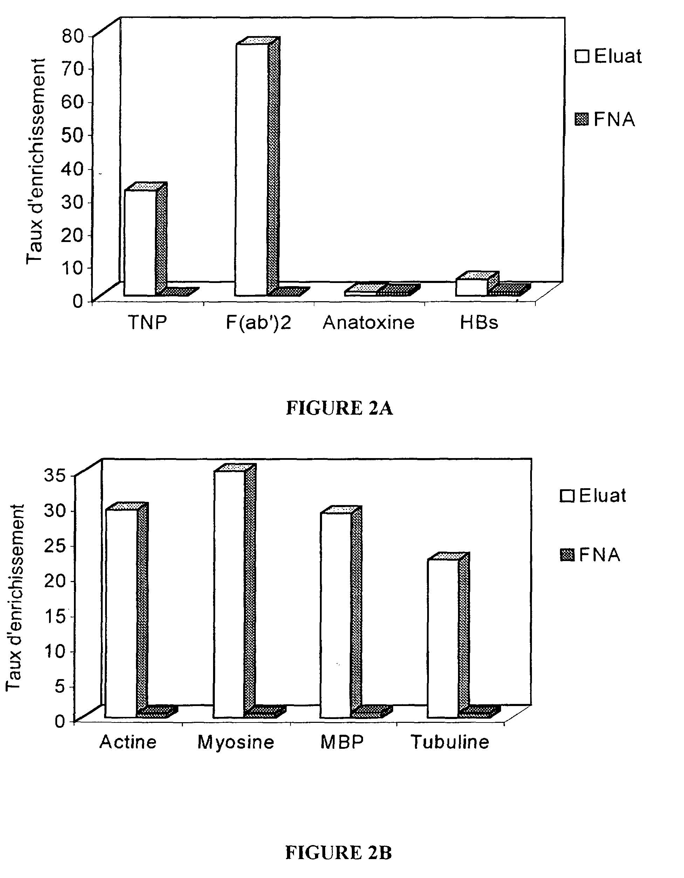

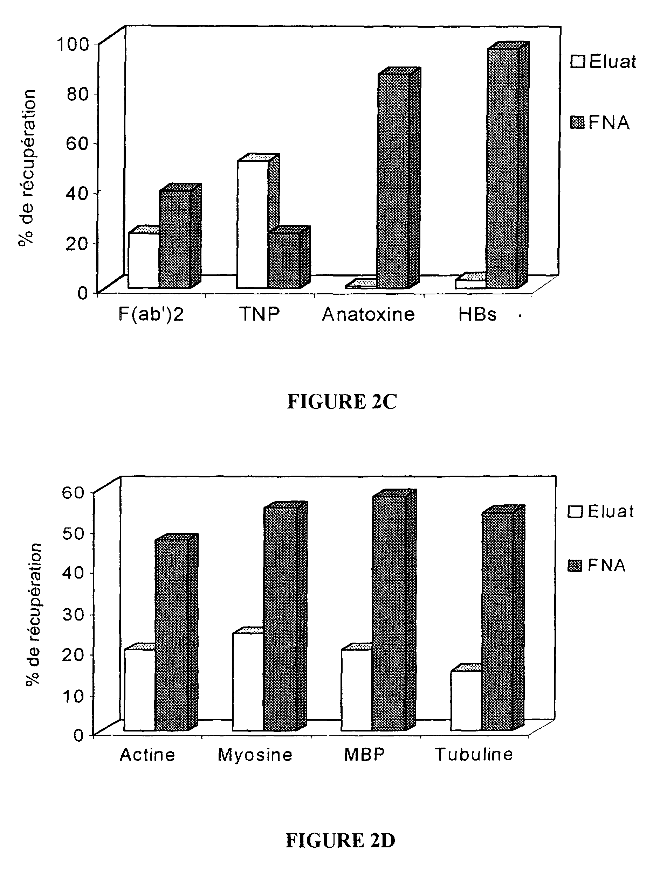

Les figures 2A et 2C illustrent la réactivité spécifique vis-à-vis des F(ab')2 d'IgG et les figures 2B et 2D représentent la réactivité vis-à-vis des autoantigènes.

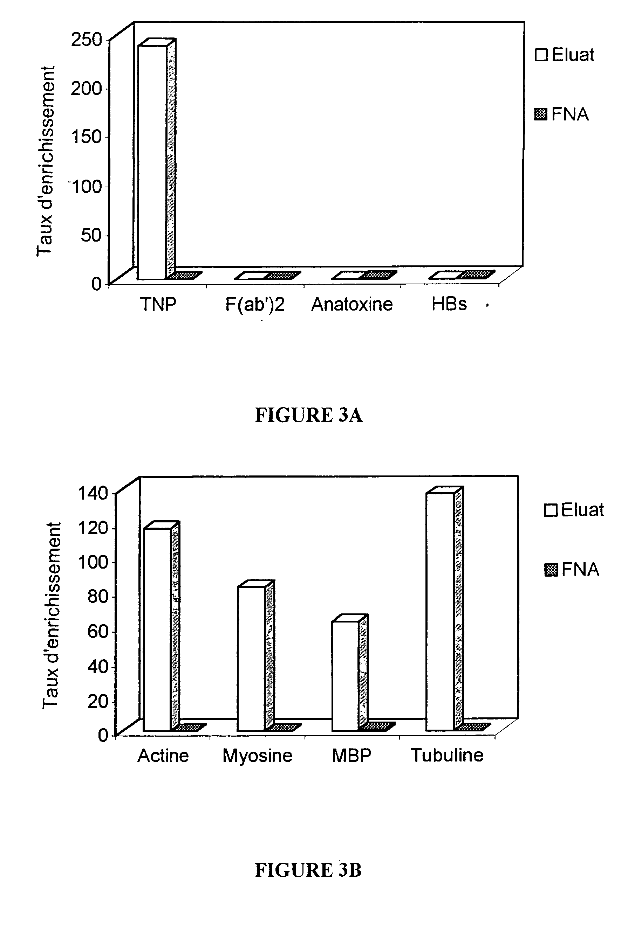

**Figure 3A-3D:** Evaluation des propriétés d'une fraction obtenue à partir de TEGELINE® (support solide à base de NHS-AffiPrep avec DNP-Lysine).
Les paramètres du procédé de préparation de cette fraction sont explicités plus en détail à l'exemple 3 ci-après.

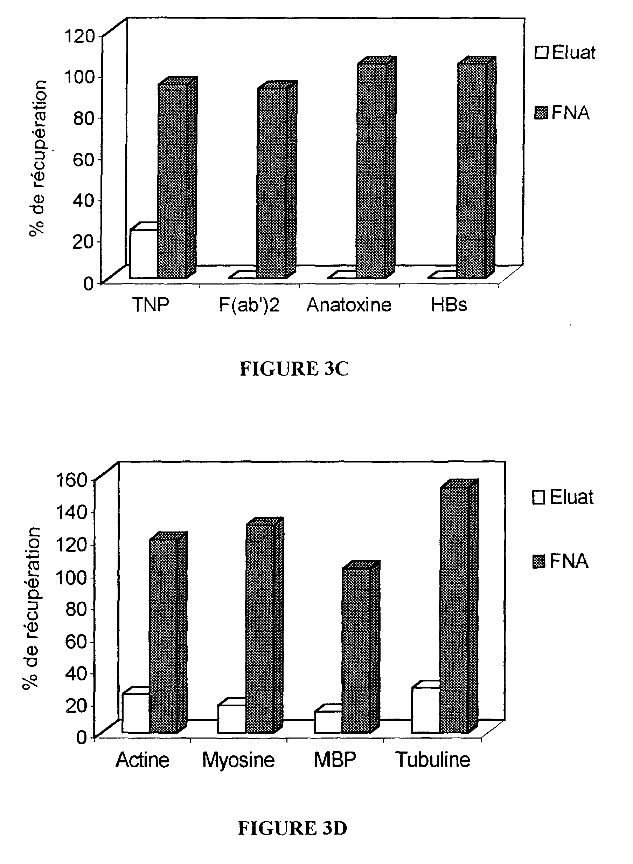

Les figures 3A et 3C illustrent la réactivité spécifique vis-à-vis des F(ab')2 d'IgG et les figures 3B et 3D représentent la réactivité vis-à-vis des autoantigènes.

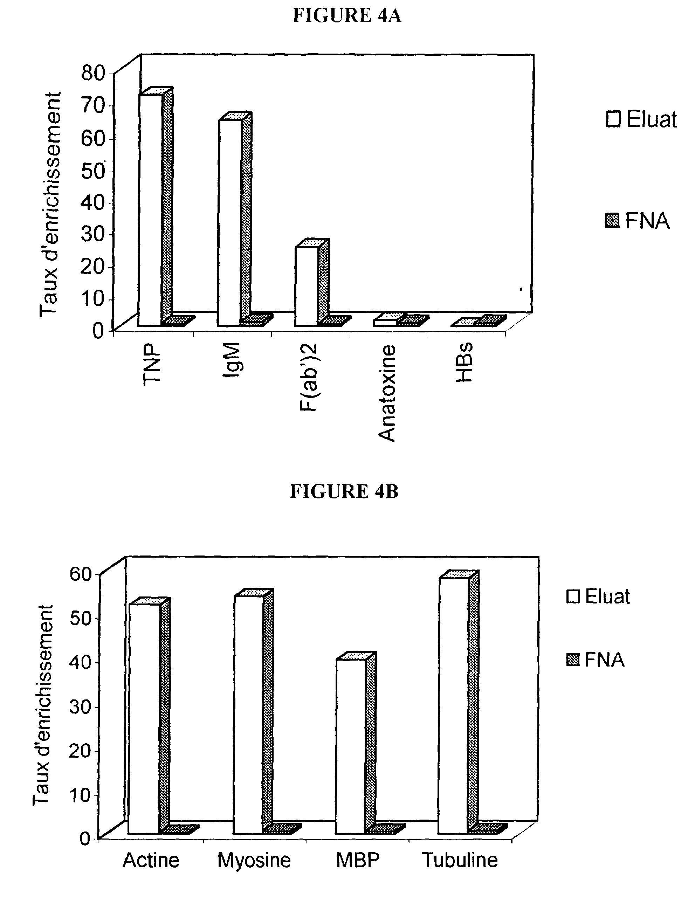

**Figure 4A-4D** : Evaluation des propriétés d'une fraction obtenue à partir de TEGELINE® (support solide à base de NHS-Sepharose greffé avec des IgM).
Les paramètres du procédé de préparation de cette fraction sont explicités plus en détail à l'exemple 4 ci-après.

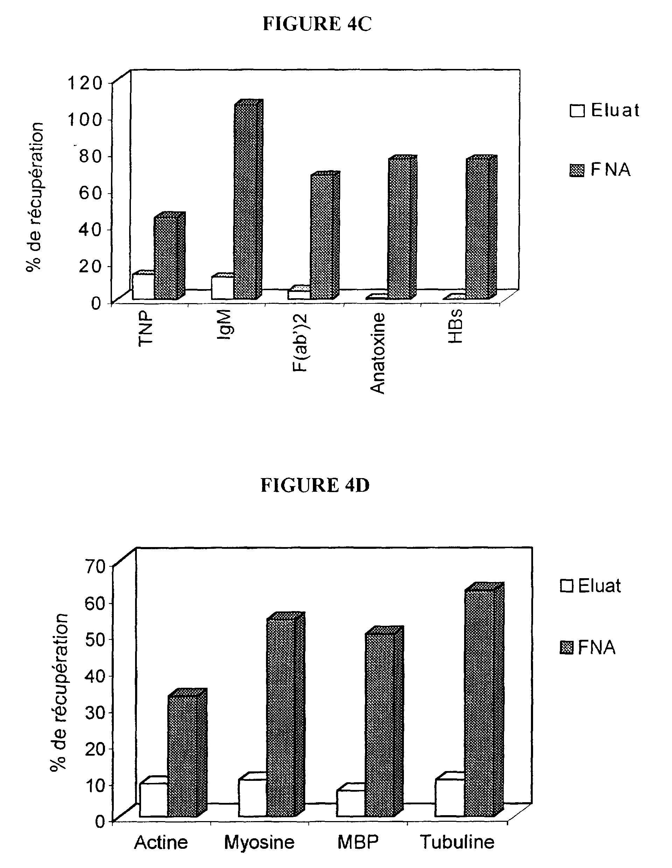

Les figures 4A et 4C illustrent la réactivité spécifique vis-à-vis des IgM et les figures 4B et 4D représentent la réactivité vis-à-vis des autoantigènes.

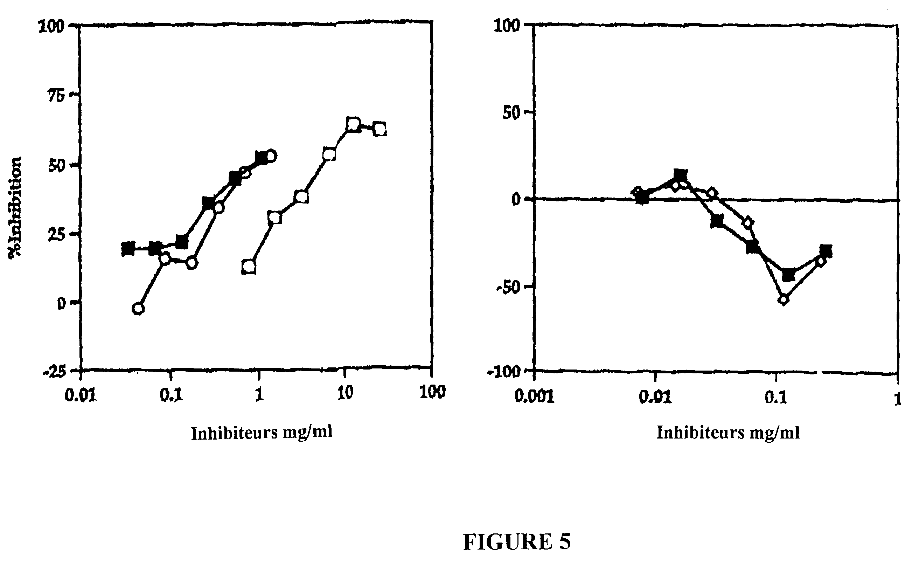

**Figure 5** : Evaluation de la capacité de TEGELINE® ou des fractions à inhiber la liaison entre l'ADN et des anticorps anti-ADN provenant d'un sérum de patient atteint d'un Lupus Erythémateux .
Les conditions expérimentales du test de compétition sont explicitées à l'exemple 6 ci-après :
■ 47-2 EN (anti-DNP) ; ○ 47-4 EN (anti-DNP) ; □ Tégéline ; ○ 46-8 EA (anti-Tégéline) ; ■ 46-9 EA (anti-Tégéline).

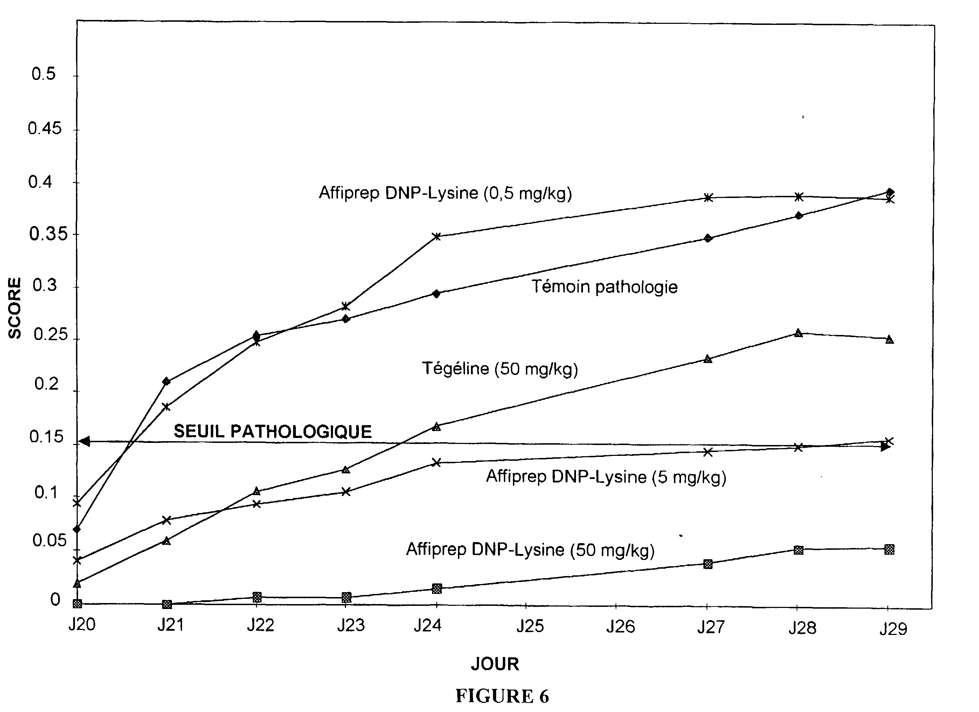

**Figure 6 :** Evaluation de l'effet protecteur de la fraction anti-DNP comparé à Tégéline sur le développement de la polyarthrite rhumatoïde induite par le collagène II chez le rat.
Cette figure représente l'évolution du score arthritique avec des fractions DNP-LYSINE.
Les modalités d'induction de la maladie ainsi que de l'administration des produits sont décrits à l'exemple 7A ci-après.

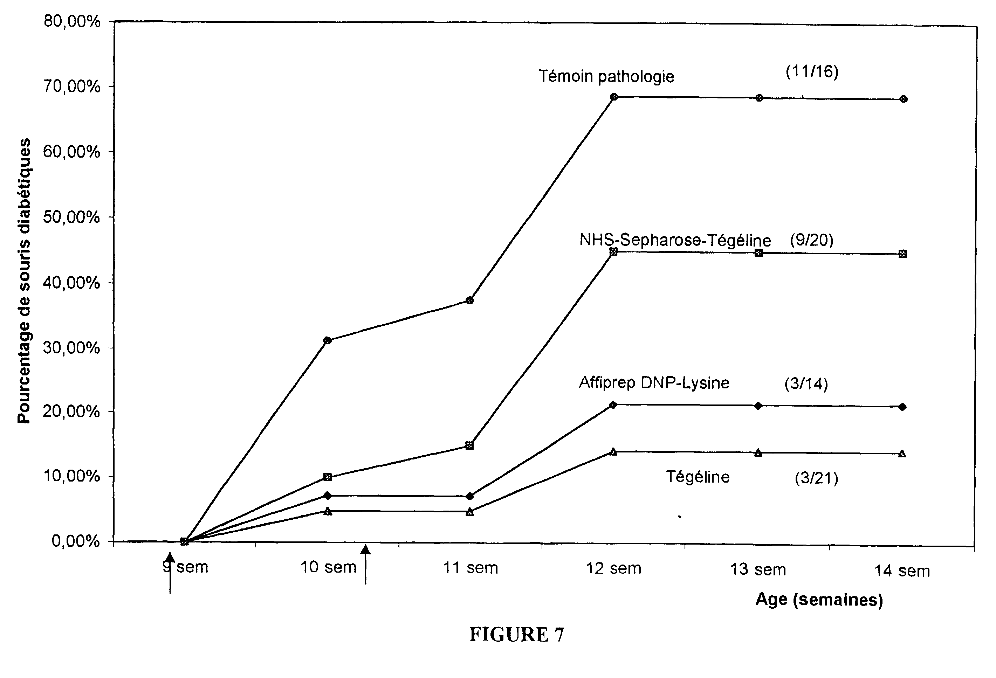

**Figure 7 ::** Evaluation de l'effet protecteur de la fraction anti-DNP comparé à Tégéline sur le développement du diabète induit par le cyclophosphamide chez la souris NOD male.
Les modalités d'induction de la maladie ainsi que de l'administration des produits sont décrits à l'exemple 7B ci-après .

Les procédés de préparation et d'évaluation de l'activité de fractions enrichies en IgG présentant la propriété de s'associer à d'autres IgG dans des interactions de type idiotypique sont présentés plus en détails dans les exemples ci-après.

### Exemple 1 : Procédé selon l'invention avec TEGELINE® et un support solide Affigel.

Les IgG polyvalentes ont été couplées à un gel de NHS-Affigel à raison de 21mg de produit par ml de gel. Une dose de 20 g d' IgG polyvalentes à la concentration de 20 mg/ml a été mise en contact par recirculation en colonne avec 21 d'immunoabsorbant pendant 4h à 22°C en PBS. L'élution a été ensuite effectuée en glycine HCL 0,1M pH 3,25 et l'éluat concentré sur une membrane d'ultrafiltration ayant un seuil de coupure de 30kD.
La concentration a été mesurée en néphélémétrie. Le taux de récupération s'élève à 0,42 % dans l'éluat et à 89 % dans la FNA.
Le taux d'enrichissement en réactivité vis à vis des F(ab')2 de cet éluat par rapport aux IgG polyvalentes de départ, s'élève à 65.

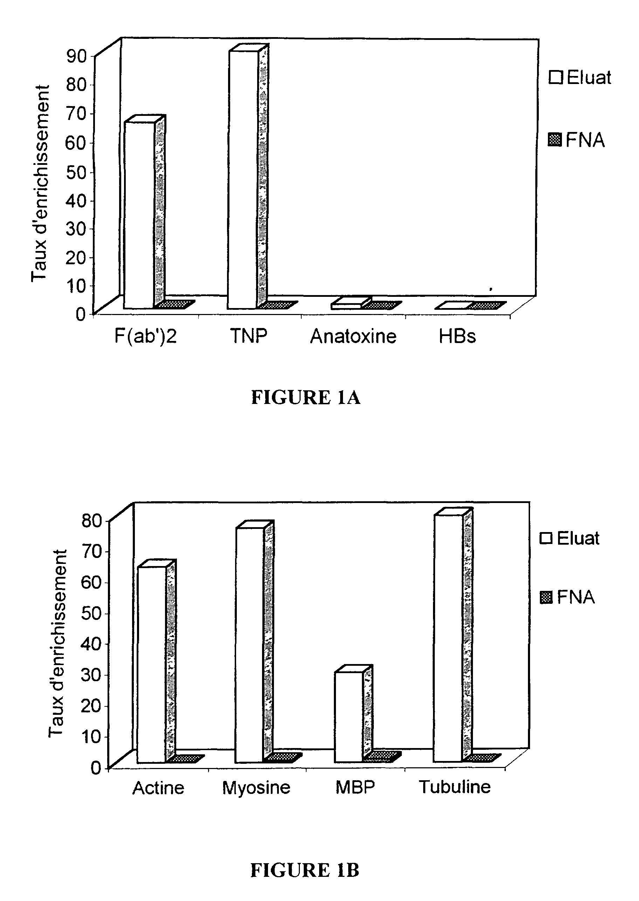

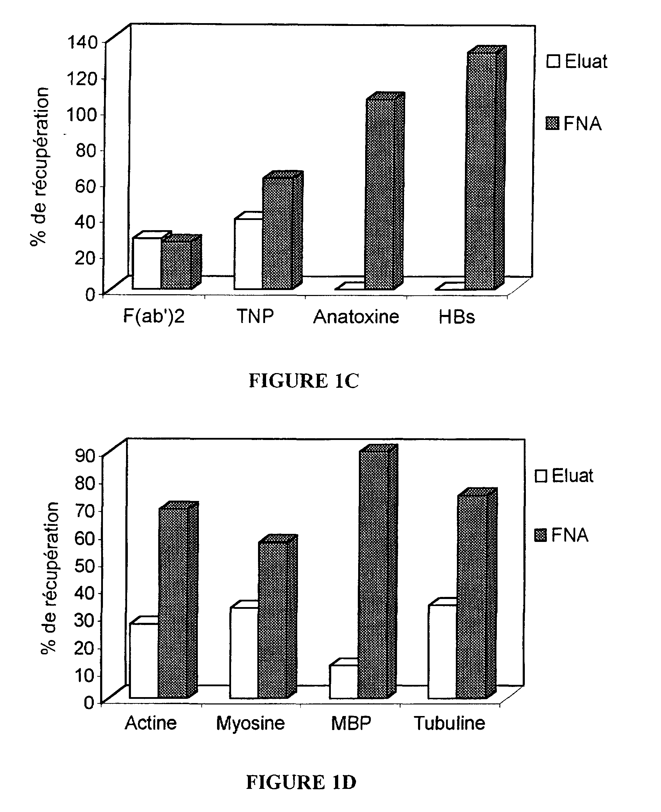

Cette fraction possède une réactivité enrichie par rapport à celle des IgG polyvalentes vis-à-vis de plusieurs autoantigènes et une absence de réactivité vis-à-vis de l'anatoxine tétanique et de l'antigène HBs (voir figure 1 et tableau 1).

**Tableau 1**

| Antigènes testés | Taux d'enrichissement | | % de récupération | |
|---|---|---|---|---|
| | Eluat | FNA | Eluat | FNA |
| F(ab')2 | 65 | 0,3 | 28 | 26 |
| TNP | 90 | 0.7 | 39 | 62 |
| Anatoxine | 1,8 | 1.1 | 0.8 | 106 |
| HBs | 2.5 | 1.4 | 1.1 | 132 |
| Actine | 63.5 | 0.7 | 27 | 69 |
| Myosine | 76 | 0,6 | 33 | 57 |
| MBP | 29 | 1 | 12 | 90 |
| Tubuline | 80 | 0.8 | 34 | 74 |

### Exemple 2 : Procédé selon l'invention avec TEGELINE® et un support solide NHS-Sepharose.

Des IgG polyvalentes ont été couplées à un gel de NHS-Sépharose à raison de 10 mg de protéine par ml de gel. Une dose de 50 mg d'IgG polyvalentes à la concentration de 1 mg/ml a été mise en contact par recirculation en colonne avec 20 ml d'immunoabsorbant pendant 4h à 22°C en PBS. La fraction non absorbée ou FNA a été recueillie et conservée à -80°C. L'élution a ensuite été effectuée en tampon glycine HCI 0,1M pH 3.5 et l'éluat concentré par centrifugation sur membrane d'ultrafiltration ayant un seuil de coupure de 30 kD. La concentration en IgG a été mesurée par néphélémétrie. Le taux de récupération s'élève à 0,77 % dans l'éluat et à 94,7 % dans la FNA.
Le taux d'enrichissement en réactivité vis-à-vis des F(ab')2 de cet éluat par rapport aux IgG polyvalentes de départ, s'élève à 76.
Cette fraction possède une réactivité enrichie par rapport à celle des IgG polyvalentes vis-à-vis de plusieurs autoantigènes et une absence de réactivité vis-à-vis de l'anatoxine tétanique et de l'antigène HBs (figure 2 et tableau 2).

**Tableau 2**

| Antigènes Testés | Taux d'enrichissement | | % de récupération | |
|---|---|---|---|---|
| | Eluat | FNA | Eluat | FNA |
| TNP | 32 | 0.45 | 22 | 39 |
| F(ab')2 | 76 | 0,2 | 51 | 22 |
| Anatoxine | 1,3 | 1 | 0,9 | 86 |
| HBs | 4,87 | 1,1 | 3,3 | 96,4 |
| Actine | 29,5 | 0,6 | 20 | 47 |
| Myosine | 35 | 0,6 | 24 | 55 |
| MBP | 29 | 0,7 | 20 | 58 |
| Tubuline | 22,4 | 0,6 | 15 | 54 |

### Exemple 3 : Procédé selon l'invention avec DNP-Lysine et un support solide NHS-AffiPrep.

Le DNP-Lysine a été couplé à un gel de NHS-Affiprep à raison de 4mg de produit par ml de gel. Une dose de 60 g d' IgG polyvalentes à la concentration de 50 mg/ml a été mise en contact par recirculation en colonne avec 2 l d'immunoabsorbant pendant 4h à 22°C en PBS. L'élution a été ensuite effectuée en iodure de sodium (Kl) 2M à pH 7. Après concentration sur une membrane d'ultrafiltration ayant un seuil de coupure de 30kD, l'éluat est déssalé contre du PBS sur une colonne de Sephadex G 25.
La concentration a été mesuré en néphélémétrie. Le taux de récupération s'élève à 0,12% dans l'éluat et à 85 % dans la FNA.
Le taux d'enrichissement en réactivité vis à vis du TNP-Ova de cet éluat par rapport aux lgG polyvalentes de départ, s'élève à 239.
Cette fraction possède une réactivité enrichie par rapport à celle des lgG polyvalentes vis-à-vis de plusieurs autoantigènes et une absence de réactivité vis-à-vis de l'anatoxine tétanique et de l'antigène HBs (figure 3 et Tableau 3).

**Tableau 3**

| Antigènes testés | Taux d'enrichissement | | % de récupération | |
|---|---|---|---|---|
| | Eluat | FNA | Eluat | FNA |
| TNP | 239 | 0,9 | 23 | 94 |
| F(ab')2 | 2,9 | 0,9 | 0,6 | 92 |
| Anatoxine | 2,4 | 1 | 0,5 | 104 |
| HBs | 3,2 | 1 | 0,7 | 104 |
| Actine | 117 | 1,1 | 24 | 120 |
| Myosine | 83 | 1,2 | 17 | 129 |
| MBP | 63 | 1 | 13 | 102 |
| Tubuline | 137 | 1,5 | 28 | 152 |

### Exemple 4 : Procédé selon l'invention avec des IgM polyclonales et un support solide NHS-Sepharose.

Des IgM polyclonales humaines (pureté 90%) ont été couplées à un gel de NHS-Sepharose à raison de 10 mg de protéines par ml de gel. Une dose de 50 mg d'lgG polyvalentes à la concentration de 1 mg/ml a été mise en contact avec 20 ml d'immunoadsorbant pendant 4h à 22°C en PBS. La fraction non adsorbée ou FNA a été recueillie et conservée à -80°C. L'élution a ensuite été effectuée en tampon glycine HCL 0,1M pH 3,5 et l'éluat concentré par centrifugation sur membrane d'ultrafiltration ayant un seuil de coupure de 30 kDa.
La concentration en IgG a été mesurée par néphélémétrie. Le taux de récupération s'élève à 0,20 % dans l'éluat et à 98,7 % dans la FNA.
Le taux d'enrichissement en réactivité vis-à-vis des IgM de cet éluat par rapport aux IgG polyvalentes de départ, s'élève à 64.
Cette fraction possède une réactivité enrichie par rapport à celle des IgG polyvalentes vis-à-vis de plusieurs autoantigènes et une absence de réactivité vis-à-vis de l'anatoxine tétanique et de l'antigène HBs (figure 4 et tableau 4).

**Tableau 4**

| Antigènes testés | Taux d'enrichissement | | % de récupération | |
|---|---|---|---|---|
| | Eluat | FNA | Eluat | FNA |
| TNP | 71,5 | 0,5 | 13 | 44,5 |
| IgM | 64 | 1,2 | 11,4 | 106 |
| F(ab')2 | 24,5 | 0,7 | 4,5 | 67 |
| Anatoxine | 1,8 | 0,8 | 0,3 | 76 |
| HBs | < seuil | 0,8 | < seuil | 76 |
| Actine | 52 | 0,3 | 9 | 33 |
| Myosine | 54 | 0,6 | 10 | 54 |
| MBP | 39,5 | 0,5 | 7 | 50 |
| Tubuline | 58 | 0,7 | 10 | 62 |

### Exemple 5 : Inhibition de la prolifération de lymphocytes humains en MLC

Les lymphocytes d'un donneur A et d'un donneur B incompatibles au niveau des molécules HLA ont été séparés sur ficoll et mis en culture à la concentration de 2X 10⁵ par puits en milieu PPM1 1640 additionné de 10 % de serum de veau foetal. Des concentrations décroissantes de Tégéline, de fragments Fc ou F(ab') 2 de Tégéline ou des différentes fractions présentées dans les exemples 1 à 4 sont ajoutées au milieu. Après 4 jours de culture à 37°C en atmosphère CO₂, 1 µCi = 37 KBq de thymidine Tritiée est ajouté pendant les dernières 6h de culture. La mesure du taux d'incorporation de thymidine tritiée dans les cellules humaines qui reflète la prolifération est effectuée à l'aide d'un compteur à scintillation β. Le pourçentage d'inhibition de la prolifération des lymphocytes en presence des différents composants ajoutés à la culture est calculé par rapport à la prolifération des cellules des donneurs A et B mélangées. Le tableau 5 presente les résultats en terme de dose en µg/ml de fractions ou de produits capables de donner une inhibition de 50% de la prolifération des cellules. Les fractions présentées dans les exemples 1 à 4 sont capbles d'inhiber la prolifération des lymphocytes en culture mixte avec une efficacité 10 à 50 fois supérieure à celle de Tégéline.

**Tableau 5 :**

| **Inhibition par TEGELINE® et par les fractions de la prolifération de lymphocytes humains en culture mixte** | | | |
|---|---|---|---|
| ***Référence fractions*** | *Support d'affinité* | ***Dose en µg*****/*****ml donnant*** *50 % d'inhibition de la prolifération* | |
| | | ***Expérience 1*** | ***Expérience 2*** |
| Tégéline® | NA | 160 | 80 |
| Fc de Tégéline® | NA | 1 000 | NT |
| F(ab')2 de Tégéline® | NA | NT | 250 |
| Exemple 1 | AffiGel NHS Tégéline | 7 | NT |
| Exemple 2 | Sepharose NHS Tégéline | 5 | NT |
| Exemple 3 | AffiPrep NHS DNP-Lysine | 9 | 2 |
| Exemple 4 | Sepharose NHS IgM | 2,5 | - |
| NA = non applicable | | | |
| NT = non testé | | | |

### Exemple 6 : Test de compétition des fractions vis à vis d'anticorps pathogènes

Tégéline ou les fractions anti-Tégéline préparées selon l'exemple 2 ou les fractions anti-DNP préparées selon l'exemple 3 sont incubées, en présence d'anticorps biotinylés anti-ADN provenant d'un malade atteint de Lupus Erythémateux, dans une plaque de microfiltration recouverte d'ADN. Le pourçentage d'inhibition de la liaison de l'anticorps biotinylé anti-ADN à l'ADN est mesuré en fonction de la concentration de Tégéline ou des fractions ajoutées. Les résultats présentés figure 5 montrent que les fractions anti-DNP inhibent environ dix fois plus la prolifération que Tégéline pour une même concentration. Les fractions anti-Tégéline au contraire favorisent la liaison des anticorps pathogènes à l'ADN en établissant des interactions de type idiotypique.

### Exemple 7 : Applications cliniques.

Les fractions enrichies en autoréactivité et se montrant efficaces dans les modèles expérimentaux de maladies autoimmunes sont destinées à être utilisées dans le traitement de nombreuses pathologies où les IglV ont été montrées comme ayant une action thérapeutique, et en particulier les maladies autoimmunes, la GVH et le rejet de greffe après transplantation.

**Exemple 7A :** Effet des fractions anti-DNP comparé à Tégéline sur le développement de la polyarthrite rhumatoïde induite par le collagène II chez le rat.

Les fractions enrichies en autoréactivité provenant de l'élution d'IgG polyvalentes d'un gel de NHS-Affiprep couplé au DNP-Lysine (fig 3 et exemple 3) ont été injectées *ip* à différentes doses à des rats ayant reçu du collagène Il pour induire le développement d'une polyarthrite rhumatoïde. L'efficacité de protection contre la polyarthrite rhumatoïde des fractions a été comparée à celle obtenue par les mêmes doses d'IgG polyvalentes initiales. Les résultats cumulés de deux expériences indépendantes (figure 6) montrent que la dose efficace sur le développement de la polyarthrite rhumatoïde des fractions provenant de l'élution du gel de NHS-Affiprep couplé au DNP-Lysine est dix fois inférieure à la dose efficace de Tégéline.

**Exemple 7B:** Effet de la fraction anti-Tégéline et de la fraction anti-DNP sur le développement du diabète induit par la cyclophosphamide chez la souris NOD mâle.

Les souris NOD mâles nouveau-nées sont injectées trois fois par semaine pendant 4 semaines soit par Tégéline à la dose de 1mg/souriceau soit par la fraction anti-Tégéline ou la fraction anti-DNP à la dose de 0.1 mg/souriceau. Le développement du diabète est déclenché à l'âge de 8 semaines par deux injections de cyclophosphamide (200 mg/kg) espacées de deux semaines. La figure 7 montre que le pourçentage de souris diabétiques [le taux de sucre dans le sang supérieur à 3g/l) est significativement diminué dans le groupe de souris NOD injecté par la Tégéline (14 %) et dans le groupe injecté par la fraction anti-DNP (21 %) mais pas dans le groupe injecté par la fraction anti-Tégéline comparé au groupe non traité (68 %)].

Ces indications ne sont pas exclusives et pourront être étendues. Lesdites fractions sont formulées avec un véhicule pharmaceutique adapté à une administration intraveineuse, avec un conditionnement soit sous forme lyophilisée, soit sous forme liquide ou une autre voie (IP, ID, IM) selon les indications recherchées.

### REFERENCES

1. Imbach P., Barandum S., D'Apuzzo V., Baumgartner C., Hirt A., Morell A., Rossi E., Schoni M., Vert-Wagner. High dose intravenous gammaglobulin for idiopathic thrombocytopenic purpura in childhood. The Lancet, 1981, i : 1128-1231.
2. Furusho K. et al . High-dose intravenous gammaglobulin for Kawaski disease. The Lancet, 1984; 1055-1058.
3. Newburger J.W. et al. A single intravenous infusion of gammaglobulin as compared with four infusions in the treatment of acute Kawasaki syndrome. N. Engl J. Med, 1991; 324 : 1633-1639.
4. Sullivan K.M. Immunomodulation in allogeneic marrow transplantation : use of intravenous immune globulin to suppress acute graft-versus-host disease. Clin. Exp. Immunol., 1996, 104 (suppl 1) : 43-48.
5. Saoudi A., Hurez V., de Kozak Y., Kuhn J., Kaveri S.V., Kazatchkine M.D. et al. Human immunoglobulin preparations of intravenous use prevent experimental autoimmune uveoretinis. Int. Immunol., 1993; 5 (12) : 1559-1567.
6. Björkholm M. Intravenous immunoglobulin treatment in cytopenic haematological disorders J. Int. Med, 1993; 234 : 119-126
7. Sultan Y., Kazatchkine M.D., Maisonneuve P., Nydegger U. Anti-idiotypic suppression of auto-antibodies to factor VIII (anti-haemophilic factor) by high-dose intravenous gammaglobulin. Lancet, 1984; 2 : 765-768.
8. Schwartz R.S., Gabriel D.A., Aledort L.M., Green D. and Kessler C.M. A prospective study of treatment of acquired (autoimmune) factor VIII inhibitors with high-dose intravenous gammaglobulin. Blood, 1995; 86 (2) : 797-804.
9. Coulam C.B., Krysa L., Strern J.J. and Bustillo M. Intravenous immunoglobulin for treatment of recurrent pregnancy loss. American Journal of Reproductive Immunology, 1995; 34 : 333-337.
10. Raziel A., Herman A., Bukovsky I., Caspin E. and Ronel R. Intravenous immunoglobulin treatment of pregnant patients with unexplained recurrent abortions. Human reproduction, 1996;11(4) : 711-715.
11. Van der Meché F.G.A., Schmitz P.I.M. and the dutch Guillain-Barré study group. A randomized trial comparing intravenous immune globulin and plasma exchange in Guillain-Barré syndrome. N. Engl. J. Med, 1992; 326 : 1123-1129.
12. Plasma exchange/Sandoglobulin Guillain-Barré syndrome trial group. Randomised trial of plasma exchange, intravenous immunoglobulin and combined treatments in Guillain-Barré syndrome. Lancet, 1997; 349 : 225-230.
13. Abdallah S.A., Jansen P.W., Ashwal S., Perkin R.M. Intravenous immunoglobulin as therapy for pediatric Guillain-Barré syndrome. J. Child. Neurol, 1997; 12 : 376-380.
14. Hahn A.F., Bolton C.F., Zochodne D. and Feasby T.E. Intravenous immunoglobulin treatment in chronic inflammatory demyelinating polyneuropathy. A double blind, placebo controlled, cross-over study. Brain, 1996; 119 : 1067-1077.
15. Dalakas M.C., Illa I., Dambrosia J.M., Soueidan S.A., Stein D.P., Otero C. et al. A controlled trial of high-dose intravenous immune globulin infusions as treatment of dermatomyositis. N. Engl. J. Med, 1993; 329 : 1993-2000.
16. Gajdos P., Chevret S., Clair B., Tranchant C., Chastang C. Clinical trial of plasma exchange and high-dose intravenous immunoglobulin in myasthenia gravis. Ann. Neurol, 1997; 41 : 789-796.
17. Fazekas F., Deisenhammer F., Stasser-Fuchs S., Nahler G. and Mamoli B. Randomised placebo controlled trial of monthly intravenous immunoglobulin therapy in relapsing remitting multiple sclerosis. Lancet, 1997, 349 : 589-593.
18. Mouthon L., Kaveri S.V., Spalter S.H., Lacroix-Desmazes S., Lefranc C., Desai R., Kazatchkine M.D. Mechanisms of action of intravenous immunoglobulin in immune-mediated diseases. Clin. Exp. Immunol, 1996; 104 (suppl 1) : 3-9.
19. Kaveri S., Dietrich G., Kazatchkine M. Can intravenous immunoglobulin treatment regulate autoimmune responses. Seminars in Hematol., 1992 ; 29 : 64.
20. Ronda N., Haury M., Nobrega A., Coutinho A., Kazatchkine M. Selectivity of recognition of variable (V) regions of autoantibodies by intravenous immunoglobulin (IVIg). Clin. Immunol. Immunopathol., 1994 ; 70 : 124.
21. Hurez V., Kaveri S. V. and Kazatchkine M. D., Expression and control of the natural autoreactive IgG repertoire in normal human serum. Eur. J. Immunol. 1993. 23 : 783-789.
22. Rossi F., Kazatchkine M. Anti-idiotype against autoantibodies in pooled normal human polyspecific Ig. J. Immunol., 1989 ; 143 : 4104.
23. Hurez V., Kazatchkine M. D., Vassilev T., Ramanathan S., Pashov A., Basuyaux B, De Kosak Y., Bellon B., Kaveri S. V. Pooled normal human polyspecific IgM contains neutralizing anti-idiotypes to IgG autoantibodies of autoimmune patients and protects from experimental autoimmune disease. Blood, 1997 ; 90 : 001
24. Berneman A., Guilbert B., Eschrich S. and Avrameas S. IgG auto- and polyreactivities of normal human sera. Molecular Immunol., 1993 ; 30 : 1499-1510.
25. Dietrich G., Kaveri S.V. and Kazatchine N.D. A V region-connected autoreactive subfraction of normal human serum immunoglobulin G. Eur. J. Immunol., 1992; 22:1701-1706.

## Revendications

1. Procédé de préparation de fractions d'Ig **caractérisé en ce qu'**il comprend les étapes suivantes :
a) Préparation d'un support insoluble sur lequel est greffé un composant sélectionné parmi les IgG, les IgM polyvalentes et du DNP-Lysine,
b) adsorption d'Ig polyvalentes sur le support obtenu à l'étape a),
c) élution des Ig retenues sur la partie des immunoglobulines liées au support de façon à recueillir la fraction connectée par interactions idiotypiques IgG-IgG ou IgM-IgG ; ou élution de la fraction interagissant avec le DNP.
d) sélection des fractions présentant i) une réactivité vis-à-vis des IgM, des F(ab')2 d'IgG ou de l'haptène DNP, ii) pas ou peu de réactivité vis-à-vis d'antigènes du non-soi, et/ou iii) une polyréactivité avec au moins l'un des autoantigènes sélectionnés parmi la myosine, l'actine, la tubuline, et la protéine basique de la myéline (MBP), i) et iii) étant **caractérisées par** une réactivité enrichie des fractions par rapport à celle des IgG polyvalentes initiales selon un taux d'enrichissement supérieur à 20 et 10 respectivement.
e) sélection des fractions par un test d'inhibition de la réaction lymphocytaire mixte avec des cellules humaines pour contrôler la réactivité des Ig purifiées.

2. Procédé selon la revendication 1 **caractérisé en ce que** les Ig absorbées consistent en des IgG ou des IgM.

3. Procédé selon l'une des revendications 1 et 2 **caractérisé en ce que** les fractions d'Ig sont préparées à partir d'Ig polyvalentes ou toute autre fraction intermédiaire obtenue au cours du procédé de fabrication des IgIV à usage thérapeutique.

4. Procédé selon la revendication 3 **caractérisé en ce que** les Ig polyvalentes utilisées pour préparer les fractions consistent en des IgG ou des IgM.

5. Procédé selon l'une des revendications 1 à 4 **caractérisé en ce que** l'étape d) comprend une mesure du taux d'enrichissement en anticorps réactifs contre les IgM, les F(ab')2 d'IgG ou l'haptène DNP utilisés pour la purification.

6. Procédé selon l'une des revendications 1 à 5 **caractérisé en ce que** l'étape d) comprend une mesure de la réactivité pour l'anatoxine tétanique et l'antigène HBs en prenant le taux d'enrichissement comme valeur contrôle.

7. Procédé selon l'une des revendications 1 à 6 **caractérisé en ce que** l'étape d) comprend un test de compétition pour contrôler l'activité neutralisante des fractions anti-DNP vis-à-vis d'anticorps biotimylés anti-ADN provenant d'un malade atteint de Lupus Erythémateux, le pourcentage d'inhibition de la liaison de l'anticorps biotimylé anti-ADN à l'ADN étant mesuré en fonction de la concentration au Tégéline.

8. Procédé selon l'une des revendications 1 à 7 **caractérisé en ce que** l'étape a) consiste en un greffage d'IgG, d'IgM polyvalentes ou de DNP-Lysine sur un support solide, notamment sur un gel de Sepharose®, de Trisacryl®, d'Affiprep®^{,} d'Affigel®, gels activés avec CNBr, NHS ou C₅H₈O₂ (glutaraldéhyde).

9. Procédé selon l'une des revendications 1 à 8 **caractérisé en ce que** les IgG déposées sur le support solide obtenu à l'étape a) sont adsorbées soit sous forme d'IgG polyvalentes lyophilisées et remises en solution ou sous forme liquide, soit sous forme de fractions intermédiaires obtenues au cours d'un procédé de fabrication d'IgG polyvalentes en tampon phosphate 20 mM comprenant du NaCl dont la concentration peut varier de 0 M à 3 M.

10. Procédé selon l'une des revendications 1 à 9 **caractérisé en ce que** l'élution des Ig retenues à l'étape b) est effectuée avec un tampon d'ions dissociant la liaison Ag-Ac ou Ag-DNP, sélectionnés notamment parmi les chaotropes tels que la glycine-HCl ou l'iodure de sodium (Nal), dans des conditions faisant varier le pH, de préférence entre 2.8 à 4.0 et/ou la molarité du tampon.

11. Procédé selon l'une des revendications 1 à 10 **caractérisé en ce que** l'absorption est effectuée dans des conditions de température allant de 4° à 40°C et en PBS.

12. Fractions susceptibles d'être obtenues à partir d'un procédé selon l'une des revendications 1 à 11 **caractérisées en ce qu'**elles réagissent avec au moins l'un des autoantigènes sélectionnés parmi la myosine, l'actine, la tubuline, et la protéine basique de la myéline (MBP) avec un taux d'enrichissement supérieur à 10 par rapport à l'activité des Ig polyvalentes initiales.

13. Utilisation d'une fraction d'Ig selon la revendications 12 pour la préparation d'un médicament.

14. Utilisation selon la revendication 13 pour la préparation d'un médicament destiné au traitement des maladies autoimmunes, de la GVH, et/ou du rejet de greffe après transplantation.

15. Utilisation selon la revendication 13 pour la préparation d'un médicament destiné au traitement de la maladie de Kawasaki, au traitement de la rétinochoroïdite de Birdshot, éventuellement en association avec une corticothérapie, au traitement de certaines cytopénies, des hémophilies avec inhibiteurs (autoanticorps anti-facteur VIII), et/ou pour prévenir et/ou empêcher le rejet immunitaire de greffes de cellules et/ou d'organes et le développement de la GVH après la greffe de cellules hématopoïétiques allogéniques.

16. Utilisation selon la revendication 13 pour la préparation d'un médicament destiné au traitement de maladies neurologiques, notamment la maladie de Guillain-Barré de l'adulte, les polyneuropathies inflammatoires chroniques démyelinisantes, les dermatomyosites, la myasthénie, et/ou la sclérose en plaques.

## Patentansprüche

1. Verfahren zur Herstellung von Ig-Fraktionen, **dadurch gekennzeichnet, dass** es die folgenden Schritte umfasst :
a) Herstellung eines unlöslichen Trägers, auf den eine Verbindung gepfropft ist, die aus polyvalenten IgG, IgM und DNP-Lysin ausgewählt ist,
b) Adsorption von polyvalenten Ig auf dem im Schritt a) erhaltenen Träger,
c) Elution der Ig, die auf dem Teil der Immunglobuline zurückgehalten sind, welche mit dem Träger verbunden sind, auf solche Weise, dass die Fraktion gewonnen wird, die durch idiotypische IgG-IgG- oder IgM-IgG-Wechselwirkungen verbunden ist; oder Elution der Fraktion, die mit DNP wechselwirkt,
d) Auswahl von Fraktionen, die (i) eine Reaktivität gegenüber den IgM, den F(ab')2 von IgG oder dem Hapten DNP, ii) keine oder wenig Reaktivität gegenüber Nicht-Selbst-Antigenen und/oder iii) eine Polyreaktivität mit mindestens einem der Autoantigene aufweisen, die aus Myosin, Actin, Tubulin und basischem Myelinprotein (MBP) ausgewählt sind, wobei i) und iii) durch eine angereicherte Reaktivität der Fraktionen bezüglich derjenigen der anfänglichen polyvalenten IgG gemäß einem Anreicherungsverhältnis von mehr als 20 bzw. 10 gekennzeichnet sind,
e) Auswahl von Fraktionen durch einen Test der Inhibierung der gemischten Lymphozytenkultur mit menschlichen Zellen, um die Reaktivität der gereinigten Ig zu kontrollieren.

2. Verfahren nach Anspruch 1, **dadurch gekennzeichnet, dass** die absorbierten Ig aus IgG oder IgM bestehen.

3. Verfahren nach einem der Ansprüche 1 und 2, **dadurch gekennzeichnet, dass** die Ig-Fraktionen ausgehend von polyvalenten Ig oder jeder anderen Zwischenfraktion hergestellt werden, die im Verlauf des Verfahrens zur Herstellung der IVIg zur therapeutischen Verwendung erhalten wird.

4. Verfahren nach Anspruch 3, **dadurch gekennzeichnet, dass** die polyvalenten Ig, die zur Herstellung der Fraktionen verwendet werden, aus IgG oder IgM bestehen.

5. Verfahren nach einem der Ansprüche 1 bis 4, **dadurch gekennzeichnet, dass** der Schritt d) eine Messung des Anreicherungsverhältnisses an reaktiven Antikörpern gegen die IgM, die F(ab')2 von IgG oder das Hapten DNP umfasst, die für die Reinigung verwendet werden.

6. Verfahren nach einem der Ansprüche 1 bis 5, **dadurch gekennzeichnet, dass** der Schritt d) eine Messung der Reaktivität für das Tetanus-Anatoxin und das HBs-Antigen umfasst, indem man das Anreicherungsverhältnis als Kontrollwert nimmt.

7. Verfahren nach einem der Ansprüche 1 bis 6, **dadurch gekennzeichnet, dass** der Schritt d) einen kompetitiven Test umfasst, um die neutralisierende Aktivität von Anti-DNP-Fraktionen gegenüber biotinylierten Anti-DNA-Antikörpern, welche von einem Kranken abstammen, der an Lupus erythematodes leidet, zu kontrollieren, wobei der Prozentsatz der Inhibierung der Bindung des biotinylierten Anti-DNA-Antikörpers an DNA als Funktion der Konzentration an Tégéline gemessen wird.

8. Verfahren nach einem der Ansprüche 1 bis 7, **dadurch gekennzeichnet, dass** der Schritt a) aus einem Pfropfen von polyvalenten IgG, IgM oder von DNP-Lysin auf einen festen Träger, insbesondere auf ein Sepharose®-, Trisacryl®-, Affiprep®-, Affigel®-Gel, Gele, die mit CNBr, NHS oder C₅H₈O₂ (Glutaraldehyd) aktiviert sind, besteht.

9. Verfahren nach einem der Ansprüche 1 bis 8, **dadurch gekennzeichnet, dass** die IgG, die auf dem im Schritt a) erhaltenen feste Träger abgeschieden werden, entweder in Form von polyvalenten lyophilisierten und wieder in Lösung gegebenen IgG oder in flüssiger Form oder in Form von Zwischenfraktionen, die im Verlauf eines Verfahrens zur Herstellung von polyvalenten IgG erhalten werden, in 20 mM Phosphat-Puffer, der NaCl umfasst, dessen Konzentration von 0 M bis 3 M variieren kann, adsorbiert werden.

10. Verfahren nach einem der Ansprüche 1 bis 9, **dadurch gekennzeichnet, dass** die Elution der im Schritt b) zurückgehaltenen Ig mit einem Puffer von Ionen, welche die Bindung Ag-Ak oder Ag-DNP dissoziieren und insbesondere aus den chaotropen Substanzen, wie Glycin-HCl oder Natriumiodid (NaI), ausgewählt sind, unter Bedingungen bewirkt wird, die den pH vorzugsweise zwischen 2,8 bis 4,0 und/oder die Molarität des Puffers variieren lassen.

11. Verfahren nach einem der Ansprüche 1 bis 10, **dadurch gekennzeichnet, dass** die Absorption bei Temperaturbedingungen, die von 4 bis 40 °C reichen, und in PBS bewirkt wird.

12. Fraktionen, erhältlich ausgehend von einem Verfahren gemäß einem der Ansprüche 1 bis 11, **dadurch gekennzeichnet, dass** sie mit mindestens einem der Autoantigene, die aus Myosin, Actin, Tubulin und basischem Myelinprotein (MBP) ausgewählt sind, mit einem Anreicherungsverhältnis von mehr als 10, bezogen auf die Aktivität der anfänglichen polyvalenten Ig, reagieren.

13. Verwendung einer Ig-Fraktion nach Anspruch 12 für die Herstellung eines Medikaments.

14. Verwendung nach Anspruch 13 für die Herstellung eines Medikaments, das zur Behandlung von Autoimmunkrankheiten, der GVH-Reaktion und/oder der Transplantatabstoßung nach Transplantation bestimmt ist.

15. Verwendung nach Anspruch 13 für die Herstellung eines Medikaments, das zur Behandlung der Kawasaki-Krankheit, zur Behandlung der Birdshot-Retinochoroiditis, gegebenenfalls in Verbindung mit einer Corticotherapie, zur Behandlung gewisser Zytopenien, von Hämophilien mit Inhibitoren (Anti-Faktor VIII-Antikörpern) und/oder zur Verhütung und/oder Verhinderung der Immunabstoßung von Zell- und/oder Organtransplantaten und der Entwicklung der GVH-Reaktion nach dem Pfropfen von allogenen hämatopoetischen Zellen bestimmt ist.

16. Verwendung nach Anspruch 13 für die Herstellung eines Medikaments, das zur Behandlung von neurologischen Krankheiten, insbesondere der Guillain-Barré-Krankheit des Erwachsenen, von chronischen entzündlichen demyelinisierenden Polyneuropathien, Dermatomyositen, Myasthenie und/oder Plaque-Sklerose bestimmt ist.

## Claims

1. Method for preparing Ig fractions, **characterized in that** it comprises the following steps:
a) preparing an insoluble support onto which is grafted a component selected from polyvalent IgGs, polyvalent IgMs and DNP-lysin,
b) adsorbing polyvalent Igs onto the support obtained in step a),
c) eluting the Igs retained on the portion of immunoglobulins bound to the support, so as to collect the fraction connected through IgG-IgG or IgM-IgG idiotypic interactions, or eluting the fraction which interacts with DNP,
d) selecting the fractions having: i) reactivity with respect to IgMs, IgG F(ab')2s or the hapten DNP, ii) little or no reactivity with respect to non-self antigens, and/or iii) polyreactivity with at least one of the autoantigens selected from myosin, actin, tubulin and myelin basic protein (MBP), i) and iii) being **characterized by** an enriched reactivity of the fractions compared to that of the initial polyvalent of IgGs according to a level of enrichment of greater than 20 and 10, respectively,
e) selecting the fractions by an assay of inhibition of the mixed lymphocyte reaction with human cells in order to control the reactivity of the purified IgGs.

2. Method according to Claim 1, **characterized in that** the Igs absorbed consist of IgGs or IgMs.

3. Method according to either of Claims 1 and 2, **characterized in that** the Ig fractions are prepared from polyvalent Igs or any other intermediate fraction obtained during the method for producing IVIgs for therapeutic use.

4. Method according to Claim 3, **characterized in that** the polyvalent Igs used to prepare the fractions consist of IgGs or IgMs.

5. Method according to one of Claims 1 to 4, **characterized in that** step d) comprises measuring the level of enrichment of antibodies reactive against IgMs, IgG F(ab')2s or the hapten DNP used for the purification.

6. Method according to one of Claims 1 to 5, **characterized in that** step d) comprises measuring the reactivity for the tetanus toxoid and the HBs antigen, taking the level of enrichment as a control value.

7. Method according to one of Claims 1 to 6, **characterized in that** step d) comprises a competition assay in order to control the neutralizing activity of the anti-DNP fractions with respect to biotinylated anti-DNA antibodies originating from a patient suffering from Lupus erythamatosus, the percentage inhibition of the binding of the biotinylated anti-DNA antibody to the DNA being measured as a function of the concentration of Tegeline.

8. Method according to one of Claims 1 to 7, **characterized in that** step a) consists in grafting polyvalent IgGs, polyvalent IgMs or DNP-Lysine onto an insoluble support, in particular onto a Sepharose®, Trisacryl®, Affiprep® or Affigel® gel, or gels activated with the groups CNBr, NHS or C₅H₈O₂ (glutaraldehyde).

9. Method according to one of Claims 1 to 8, **characterized in that** the Igs deposited onto the solid support obtained in step a) are adsorbed either in the form of polyvalent IgGs lyophilized and redissolved or in liquid form, or in the form of intermediate fractions obtained during a method for.producing polyvalent IgGs, in 20 mM phosphate buffer containing NaCl, the concentration of which may range from 0 M to 3 M.

10. Method according to one of Claims 1 to 9, **characterized in that** the Igs retained in step b) are eluted with a buffer containing ions which dissociate Ag-Ab or Ag-DNP binding, selected in particular from chaotropes such as glycine-HCl or sodium iodide (NaI), under conditions which vary the pH, preferably between 2.8 and 4.0, and/or the molarity of the buffer.

11. Method according to one of Claims 1 to 10, **characterized in that** the absorption is carried out under temperature conditions ranging from 4° to 40°C and in PBS.

12. Fractions which can be obtained from a method according to one of Claims 1 to 11, **characterized in that** they react with at least one of the autoantigens selected from myosin, actin, tubulin and myelin basic protein (MBP), with a level of enrichment of greater than 10 compared to the activity of the initial polyvalent Igs.

13. Use of an Ig fraction according to Claim 12, for preparing a medicinal product.

14. Use according to Claim 13, for preparing a medicinal product intended for the treatment of autoimmune diseases, or GVH and/or of graft rejection after transplantation.

15. Use according to Claim 13, for preparing a medicinal product intended for the treatment of Kawasaki disease, for the treatment of Birdshot retinochoroiditis, optionally in combination with corticotherapy, and/or for the treatment of certain cytopenias and/or of haemophilias with inhibitors (anti-factor VIII autoantibodies), and/or for preventing and/or impeding immune rejection of cell and/or organ transplants and the development of GVH after transplantation of allogenic haematopoietic cells.

16. Use according to Claim 13, for preparing a medicinal product intended for the treatment of neurological diseases, in particular adult Guillain-Barré syndrome, chronic demyelinating inflammatory polyneuropathies, dermatomyositis, myasthenia and/or multiple sclerosis.
